(19) Europäisches Patentamt / European Patent Office / Office européen des brevets

(11) EP 3 779 993 A1

(12) **EUROPEAN PATENT APPLICATION**
published in accordance with Art. 153(4) EPC

(43) Date of publication:
**17.02.2021 Bulletin 2021/07**

(51) Int Cl.:
**G16B 40/00** (2019.01)      **G16B 25/20** (2019.01)
**G16B 5/20** (2019.01)

(21) Application number: **19799534.3**

(22) Date of filing: **08.05.2019**

(86) International application number:
**PCT/KR2019/005499**

(87) International publication number:
**WO 2019/216643 (14.11.2019 Gazette 2019/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.05.2018 KR 20180052522**

(71) Applicant: **Deep Bio Inc.**
**Seoul 08394 (KR)**

(72) Inventor: **KIM, Sun Woo**
**Seongnam-si Gyeonggi-do 13599 (KR)**

(74) Representative: **Flügel Preissner Schober Seidel Patentanwälte PartG mbB**
**Nymphenburger Strasse 20**
**80335 München (DE)**

(54) **METHOD FOR SEARCHING FOR ENDPOINT OF SPECIFIC DATA CLUSTER AND DATA PROCESSING SYSTEM THEREFOR**

(57)      Disclosed are a method for searching for an endpoint of a specific data cluster and a data processing system therefor. The method for searching for an endpoint of a specific data cluster comprises the steps of: a) receiving, by a search system, an input of a numerical value for each of a plurality of individual data included in a data set; b) dividing a numerical range, to which the numerical value may belong, into a plurality of bins each having a predetermined bin width, by using the respective numerical values received by the search system, and generating histogram data having, as a bin value, the number of individual data corresponding to each of the divided bins; and c) searching for a target bin present at an endpoint of a specific cluster on the basis of the histogram data generated by the search system.

FIG. 3

O : 1.23425, 2.13425, 4.23252, 3.13141, 1.14452 ...

EP 3 779 993 A1

## Description

[Technical Field]

The present invention relates to a method of searching for the endpoint of a specific data group and a data processing system therefor and, more particularly, to a method and system capable of effectively searching for the endpoint of a specific data group (e.g., an ending point or a starting point) in a data set having at least one data group.

[Background Art]

In many technologies or service fields, multiple data are analyzed and used.

For example, a method of determining whether to apply a medicine to each patient by applying specific medical data or applying a specified treatment method to a person is actively applied.

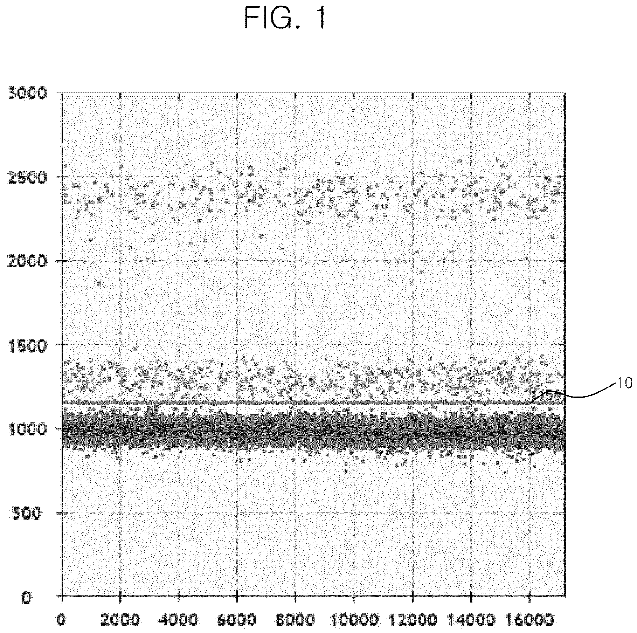

FIG. 1 illustrates an example of medical data used in a given companion diagnostic kit, that is, means for selecting a patient suitable for a given targeted anticancer agent.

FIG. 1 is an embodiment and is medical data obtained using a test kit (e.g., GenesWell ™ ddEGFR Mutation Test) notifying whether a mutation is present in a specific gene (e.g., exon 18, 19, 20, 21) of EGFR, that is, a representative bio marker of lung cancer. Such medical data may be analyzed and used to select a patient who has an effect on a medicine before a targeted agent is prescribed after an operation. However, the technical spirit of the present invention is not limitedly applied to such an embodiment, and may be used in various data analysis.

Furthermore, in a graph, such as that illustrated in FIG. 1, each point indicates individual medical data. Furthermore, in an embodiment, as illustrated in FIG. 1, the individual medical data may form at least one group (e.g., three groups in FIG. 1) in coordinate values.

In this case, it may be necessary to determine an endpoint in a specific data group (e.g., at least one individual medical data present at the top of the lowest data group, that is, a first group (e.g., at least one medical data having the greatest y-axis value) or a data value (y-axis value)) of such individual medical data.

However, it is difficult to be aware to which group which individual medical data belongs based on only each individual medical data, based on only data numerical values or coordinate locations indicated in coordinate values. In particular, such a difficulty may be further increased if multiple individual medical data is present between data groups.

Practically, conventionally, as illustrated in FIG. 1, a work method of checking individual medical data indicated in coordinate values by the naked eye and randomly drawing a division line 10 for checking an endpoint (e.g., a top point in the first (lowest) group) is used.

However, in such a case, there are problems in that the endpoint may be different depending on a person who performs the task and accuracy may be reduced.

[Disclosure]

[Technical Problem]

Accordingly, an object of the present invention is to provide a method and system capable of automatically searching for the endpoint of a specific data group rapidly in a data set including at least one data group.

[Technical Solution]

According to an aspect of the present invention, a method of searching for an endpoint of a specific group in a data set having at least one group includes the steps of a) receiving, by a search system, a numerical value of each of multiple individual data included in the data set; b) dividing, by the search system, a numerical range to which the numerical values are able to belong into a plurality of bins each having a predetermined bin width using the numerical values and generating histogram data having the number of individual data, corresponding to each of the divided bins, as a bin value; c) equalizing the histogram data; d) differentiating the equalized histogram data; and e) searching for a target bin satisfying a given criterion condition and present at an endpoint of a specific group based on the differentiated histogram data.

The step of searching for a target bin satisfying a given criterion condition and present at an endpoint of a specific group based on the differentiated histogram data may include the step of searching for, as the target bin, a bin satisfying the criterion condition based on the criterion condition in which a case where a value of a previous bin of a current bin now being searched for is smaller than a value of a subsequent bin, the value of the previous bin is equal to or smaller than 0, and the value of the subsequent bin is equal to or greater than 0 is a first bin, while searching the differentiated histogram data for a bin value of each of the bins in a given direction.

The method may further include the steps of f) reducing a preset bin width by a given numerical value if the target bin satisfying the criterion condition is not searched for using the bin width; and performing the steps b) to e) again using the reduced bin width.

According to another embodiment for solving the object, a method of searching for an endpoint of a specific group in a data set having at least one group includes the steps of a) receiving, by a search system, a numerical value of each of multiple individual data included in the data set; b) dividing, by the search system, a numerical range to which the numerical values are able to belong into a plurality of bins each having a predetermined bin width using the numerical values and generating histogram data having the number of individual data, corresponding to each of the divided bins, as a bin value; c) equalizing the histogram data; d) searching for a target bin satisfying a given criterion condition and present at an endpoint of a specific group based on the equalized histogram data.

The step of searching for a target bin satisfying a given criterion condition and present at an endpoint of a specific group based on the equalized histogram data may include the step of searching for, as the target bin, a bin satisfying the criterion condition based on the criterion condition in which a value of a previous bin of a current bin now being searched for is not a cutoff value, a value of the current bin is not a cutoff value, and a value of at least one next bin has a cutoff value while searching the equalized histogram data for a bin value of each of the bins in a given direction.

The methods may be implemented by a computer program installed in the data processing system.

A system for solving the object includes a processor and a memory device in which software executed by the processor is stored, wherein the software receives a numerical value of each of multiple individual data included in a data set having at least one data group, dividing a numerical range to which the numerical values are able to belong into a plurality of bins each having a predetermined bin width using the numerical values, generates histogram data having the number of individual data, corresponding to each of the divided bins, as a bin value, and searches for a target bin present at an endpoint of a specific group based on the generated histogram data.

The software may equalize the histogram data, may differentiate the equalized histogram data, and may search for the target bin, satisfying a given criterion condition and present at the endpoint of the specific group, based on the differentiated histogram data.

The software may equalize the histogram data, and may search for the target bin satisfying a given criterion condition based on the equalized histogram data.

If the target bin satisfying the criterion condition is not searched for using a preset bin width, the software may reduce the bin width by a given numerical value, may generate a histogram again using the reduced bin width, and may search for the target bin present at the endpoint of the specific group using the re-generated histogram.

[Advantageous Effects]

According to the technical spirit of the present invention, there is an effect in that the endpoint of a specific data group can be automatically searched for using numerical values of individual data even without separately clustering multiple individual data.

Accordingly, there is an effect in that an endpoint can be consistently and accurately searched for compared to the existing task that is manually performed.

[Description of Drawings]

In order to more sufficiently understand the drawings cited in the detailed description of the present invention, a brief description of each drawing is provided.

FIG. 1 is an exemplary diagram for describing a conventional method of searching for the endpoint of a specific data group.

FIG. 2 is a diagram for describing a schematic configuration of a search system according to an embodiment of the present invention.

FIG. 3 is a diagram for conceptually describing a method of searching for the endpoint of a specific data group according to an embodiment of the present invention.

FIG. 4 is a flowchart for describing a method of searching for the endpoint of a specific data group according to an embodiment of the present invention.

[Best Mode for Invention]

In order to sufficiently understand the present invention, operational advantages of the present invention, and an object achieved by carrying out the present invention, reference needs to be made to the accompanying drawings illustrating preferred embodiments of the present invention and contents described with reference to the accompanying drawings

Furthermore, in this specification, if any one element "transmits" data to the other element, this means that the element may directly transmit the data to the other element or may transmit the data to the other element through at least still another element.

In contrast, if any one element "directly transmits" data to the other element, this means that the data is transmitted from the element to the other element without the intervention of another element.

Hereinafter, the present invention is described in detail by describing a preferred embodiment of the present invention with reference to the accompanying drawings. In each drawing, the same reference numeral denotes the same member.

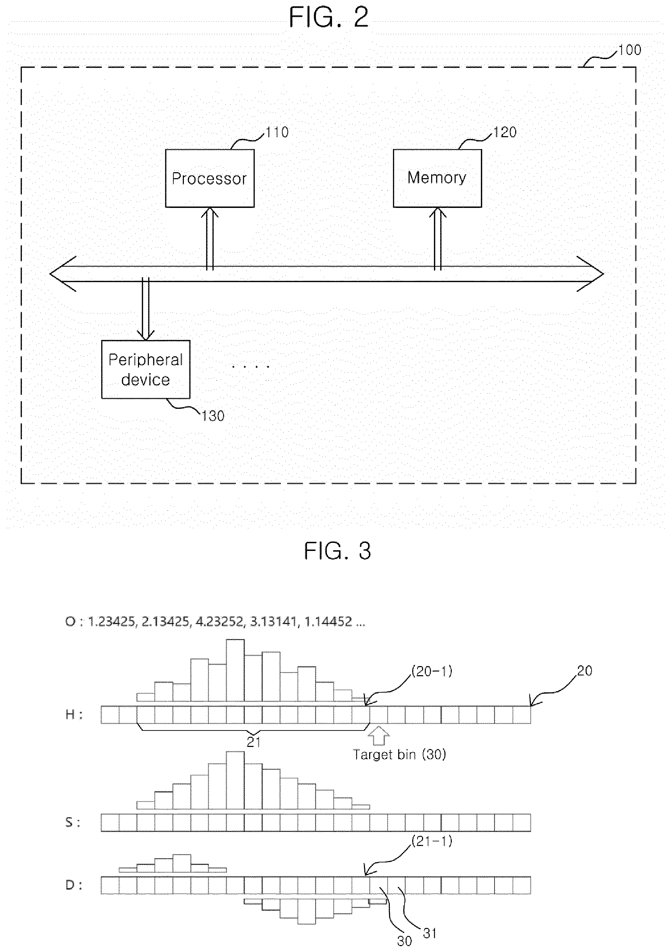

FIG. 2 is a diagram for describing a schematic configuration of a search system according to an embodiment of the present invention.

Referring to FIG. 2, the search system 100 according to the technical spirit of the present invention includes a processor 110 and a memory 120.

The memory 120 may store a computer program (software) for implementing the technical spirit of the present invention.

The software may perform a method of searching for the endpoint of a specific data group according to the technical spirit of the present invention by being driven by the processor 110.

In an embodiment, the search system 100 may include at least one of a given peripheral device 130. The peripheral device may be various, such as a display device, a speaker, an audio/video processing module, an external memory, an input and output device, and a communication device.

The search system 100 may be implemented as any data processing system having the data processing ability to search for the endpoint of a specific data group according to the technical spirit of the present invention, such as a computer, a server, or a mobile phone.

The search system 100 may receive a given data set. The data set may include multiple individual data. Each of the multiple individual data have a given value. The value may be a numerical value. Furthermore, the multiple individual data may form at least one data group.

An example according to the technical spirit of the present invention may be medical data as described with reference to FIG. 1, but the scope of the rights of the present invention is not limited thereto, and the present invention may be used for various data.

The search system 100 generates histogram data using an input data set. The histogram data has, as a domain in a first axis (e.g., x-axis), the range of numerical values to which the individual data may belong. The histogram data may include information on each bin if the first axis is divided into a plurality of bins each having a given bin width.

The numerical value to which the individual data may belong may be a positive real value, and a maximum value of the numerical value may be predetermined.

The information on each bin may have the range of a first axis value of a corresponding bin (or the index of the corresponding bin indicating what place is the corresponding bin located) and a second axis (e.g., y-axis) value of the corresponding bin. The second axis value of the bin may be the number of individual data corresponding to the range of the first axis value (i.e., a numerical value of individual data having a range corresponding to a bin width).

Furthermore, the search system 100 may search at least the endpoint (e.g., the top point) of a specific group (e.g., the first group) from at least one group based on histogram data including such information on a bin.

To search for the endpoint may mean numerical values of the first individual data (or the range of numerical values right after a numerical value of the first individual data) in order of greater numerical value (e.g., upward in the y-axis of FIG. 1), among individual data included in a specific group, or the range of numerical values of some individual data (or the range of the numerical value right after the range) in order of greater numerical value.

Or, the endpoint may mean the range of numerical values of the first individual data (or the range of numerical values of previous individual data smaller than the numerical value of the first individual data) in order of smaller numerical value (e.g., downward in the y-axis of FIG. 1), among individual data included in a specific group, or the range of numerical values to which a predetermined number (e.g., 2 to 3) of individual data (or the range of numerical values right before the range) belong in order of smaller numerical value.

The example described with reference to FIG. 1 illustrates a case where the top point of the first data group is searched for in a plurality of data groups in order of smaller numerical value, but the technical spirit of the present invention does not need to be essentially applied to the first data group or does not need to be applied to the only search for the top point of a specific data group. For example, as will be described later, if the endpoint of a specific group is searched for using histogram data, the endpoint of a given data group (e.g., the second data group) may be searched for depending on which (e.g., second) endpoint is searched for in a search direction (e.g., in a direction from a bin having a small numerical value to a bin having a great numerical value in the first axis). Furthermore, the bottom point of a specific data group not the top point thereof may be searched for in a search direction (e.g., in a direction from a bin having a great numerical value to a bin having a small numerical value in the first axis).

Hereinafter, a case where the top point of the first data group is searched for in a plurality of data groups in order of small numerical value is described as an example, for convenience of description, but the scope of the rights of the present invention is not limited thereto.

According to the technical spirit of the present invention, the endpoint of a specific data group may be searched for using histogram data. Such a case may be defined as a problem for searching the histogram data for an endpoint bin, that is, a target bin corresponding to a corresponding data group.

Furthermore, according to an embodiment, according to the technical spirit of the present invention, histogram data is used not only without any change, but also equalized histogram data and/or histogram data obtained by performing differentiation on the equalized histogram data may be used.

That is, in an embodiment of the present invention, a target bin may be searched for using equalized histogram data, and a target bin may be searched for using histogram data obtained by differentiating the equalized histogram data. Furthermore, there is an effect in that an inflection point of histogram data can be intuitively easily determined compared to a case where differentiation is performed.

The technical spirit of the present invention has effects in that it can prevent a case where individual data corresponding to a specific bin (i.e., a range near a specific numerical value) is not temporarily present if a target bin is searched for by simply transforming the original individual data into histogram data through equalizing or equalizing and differentiation and a case where if individual data is present, a specific bin is searched for as the target bin, that is, the endpoint of a specific data group to be searched.

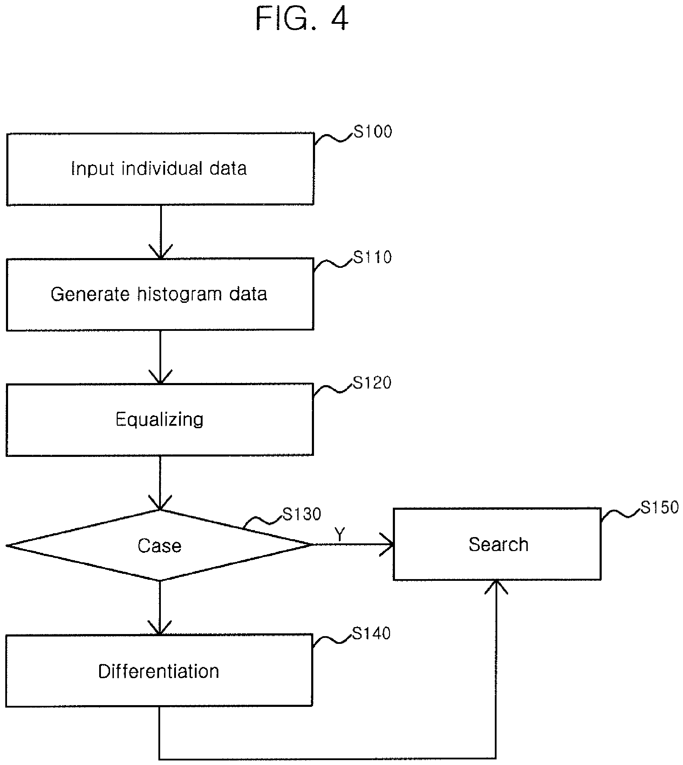

The technical spirit of the present invention is more specifically described below with reference to FIGS. 3 and 4.

FIG. 3 is a diagram for conceptually describing a method of searching for the endpoint of a specific data group according to an embodiment of the present invention. Furthermore, FIG. 4 is a flowchart for describing a method of searching for the endpoint of a specific data group according to an embodiment of the present invention.

FIG. 3 shows illustrative histogram data if the diameter of a mask (i.e., the number of parameters) is 3 and equalizing and/or differentiation is simply performed using the mask having parameter values of [-1. 0. 1], but the diameter of a equalizing mask and/or differentiation mask and the parameter values may be various.

Referring to FIGS. 3 and 4, the search system 100 may sequentially receive the original individual data O included in a data set (S100).

As illustrated in FIG. 3, the original individual data O may have positive real values (e.g., 1.23425, 2.13425, 4.23252, 3.13141, 1.14452). Such a positive real value may be a value corresponding to the second axis (e.g., the y-axis) in the graph of FIG. 1.

Accordingly, the search system 100 may generate histogram data H based on the input original individual data O (S110).

The histogram data H may be data generated by dividing the range of numerical values to which the individual data may belong into a plurality of bins 20 each having a given width so that the number of individual data having numerical values corresponding to each of the divided bins has a bin value of each of the bins.

If the histogram data H is diagrammed, it may be the same as the histogram data H of FIG. 3.

In the histogram data H of FIG. 3, bins 21 having a bin value may be a partial area of the histogram corresponding to any one data group.

Accordingly, the endpoint of a data group, that is, a target bin 30 to be searched for by the search system 100, may be the same as that illustrated in FIG. 3. That is, FIG. 3 illustrates a case where not last individual data, that is, the range of numerical values of individual data corresponding to a previous bin 21-1 of the last target bin 30 (i.e., the range of first axis values of the previous bin 21-1), but a bin right after the previous bin 21-1 having the corresponding bin value is searched for as the target bin 30. However, according to an embodiment, the previous bin 21-1 may be a target bin.

The search system 100 does not directly search the histogram data H for the target bin 30, but may equalize the histogram data H (S120).

Furthermore, the search system 100 may search for the target bin 30 using the equalized histogram data S (S150).

By using the equalized histogram data S as described above, a problem in that whether to determine such a blank bin as a target bin or a blank bin is unclear can be solved if at least one bit (called a blank bin) having a bin value of 0 is temporally present between a series of the bins 21 having the bin value, that is, if there is some range in which individual data is not present in the range of numerical values corresponding to a data group to be searched. That is, if the equalized histogram data S is used, a bin value may not be 0 because the bin has a given value depending on left and right bin values although the bin is blank bin in the original histogram H. Accordingly, to use the equalized histogram data S may be more effective.

The results of the equalizing of the original histogram data H as described above may be the same as the histogram S of FIG. 3.

A equalizing mask (or filter) and/or a differential mask for equalizing a histogram has been widely known.

In an embodiment of the present invention, a equalizing mask and/or a differential mask may use a convolution mask. A given digit string x and a convolution mask h may be defined as follows.

[Equation 1]

$$x[n]*h[n] = \sum_{k=-\infty}^{\infty} x[k]h[n-k] = \sum_{l=-\infty}^{\infty} x[n-l]h[l]$$

$$\equiv \sum_{k=-\infty}^{\infty} h[k]x[n-k] = h[n]*x[n]$$

In an embodiment of the present invention, for example, a equalizing mask and a differential mask use [1, 1, 1, 1, 1, 1, 1, 1, 1, 1] and [-1, -1, -1, -1, 0, 1, 1, 1, 1]. In another embodiment, [1, 1, 1, 1, 1, 1, 1, 1, 1, 1] and [-1, -1, -1, -1, 0, 1, 1, 1, 1] are used as a equalizing mask and a differential mask. However, the equalizing mask and the differential mask may be variously set depending on the characteristics of a data set, such as the number of individual data included in a data set, a degree of grouping, etc.

Meanwhile, as described above, the search system 100 may search for the target bin 30 using the equalized histogram data S. In another case, the target bin 30 may be more clearly searched for by differentiating the equalized histogram data S.

Whether the target bin 30 will be searched for using the equalized histogram data S in any case and whether the target bin 30 will be searched for using the differentiated histogram data D in any case may be determined depending on characteristics of a data set. The characteristics of the data set may be determined based on characteristics of the data set, including the number of data, the density of data, and the number of data groups. A criterion for searching for the target bin 30 in a first case, that is, using the equalizing histogram data S, and searching for the target bin 30 using the differentiation histogram data D in a second case, if characteristic belongs to a given range through repetitive experiments that are previously performed, may be determined.

According to an embodiment, any one of the two methods may be randomly selected. In an implementation example, the target bin 30 may be searched for using the two methods and the results of the search may be compared.

If the target bin 30 is searched for using the two methods, if locations (first axis values) of respective retrieved target bins are the same or fall within a predetermined location (first axis value) range, a target bin retrieved using any one method may be determined as the final target bin.

Accordingly, if the first case is determined (S130) based on the input original individual data O, the search system 100 may search for the target bin 30 using the equalized histogram data S as described above (S150).

Furthermore, if the search system 100 determines the second case, the search system 100 may differentiate the equalized histogram data S (S140). Accordingly, the search system 100 may search for the target bin 30 using the differentiated histogram data D (S150).

An example in which the search system 100 searches the equalized histogram data S for the target bin 30 may be as follows.

For example, the search system 100 may search the equalized histogram data S for a bin value of each of bins in a given direction (e.g., a direction in which a numerical value becomes great).

Accordingly, the target bin 30 whose value of a previous bin of a current bin now being searched for is not a cutoff value (e.g., 0), whose value of the current bin is a cutoff value (e.g., 0), and whose value of predetermined number (e.g., 1 or 2 or more) of next bin is a cutoff value (e.g., 0) may be searched for.

In such a case, in FIG. 3, if the target bin 30 is a current bin now being searched for, when a bin value of the previous bin 21-1 is not 0 and a bin value of the current bin is 0, the current bin may be determined as the target bin 30 because bin values of bins right after a predetermined number (e.g., 2) are 0.

The cutoff value may be 0, but may be set to have a small value, such as 1, according to an embodiment. In such a case, an endpoint to be searched for may be defined as an algorithm for searching a data group for a numerical value including only one individual data at the end point side. In an embodiment, the cutoff value may be variously set.

Meanwhile, an example in which the target bin 30 is searched for in the differentiated histogram data D may be as follows.

For example, the search system 100 may search the differentiated histogram data D for a bin value of each of bins in a given direction (e.g., a direction in which a numerical value becomes great).

If a current bin being searched for is the target bin 30, a case where a bin value of the previous bin 21-1 of the current bin is smaller than a bin value of a subsequent bin 31, the bin value of the previous bin 21-1 is equal to or smaller than 0, and the bin value of the subsequent bin 31 is equal to or greater than 0 may correspond to the target bin 30 to be searched for.

That is, a point corresponding to an area where a bin value is gradually increased from a negative value and becomes 0 may be the target bin 30 to be searched for.

Meanwhile, when the aforementioned histogram data is generated, the target bin 30 may not be searched for depending on how the bin width is set. For example, when the width of a bin is too large, if multiple individual data is present between a data group to be searched and a subsequent data group and is relatively crowded, a bin having a cutoff value may not be present.

If a bin width is too narrowed, there is a problem in that multiple bins having a cutoff value may be detected within one data group or there is a problem in that a search time is long because the number of bins is increased.

Accordingly, it may be necessary to previously determine a proper bin width through repetitive experiments.

If it is difficult to previously determine such a bin width, a search may be performed using a given default bin width value. If a target bin, such as that described above, is not searched for (i.e., if a bin width is wide to the extent that a bin having a bin value of 0 is not present between the endpoint bin of a target data group to be searched and the endpoint bin on the target data group of a data group neighboring the target data group), histogram data may be generated using a narrowed bin width while the bin width is sequentially narrowed by a predetermined unit value. Furthermore, the search of a target bin using the aforementioned target bin search process (the search using the equalized histogram data or the search of a target bin using the differentiated histogram data) may be performed using the generated histogram data.

In such a case, there are effects in that a bin width having relatively excellent search efficiency can be searched for and a target bin, such as that described above, can be searched for using the retrieved bin width.

Meanwhile, the method of searching for the endpoint of a specific data group according to an embodiment of the present invention may be implemented in the form of computer-readable program instructions and stored in a computer-readable recording medium. A control program and target program according to embodiments of the present invention may also be stored in a computer-readable recording medium. The computer-readable recording medium includes all types of recording devices in which data readable by a computer system is stored.

The program instructions written in the recording medium may have been specially designed or configured for the present invention or may have been known to and available by a person skilled in the software field.

Examples of the computer-readable recording medium include hardware devices specially configured to store and execute program instructions, such as magnetic media such as a hard disk, a floppy disk and a magnetic disk, optical media such as a CD-ROM and a DVD, magneto-optical media such as a floptical disk, a ROM, a RAM, and a flash memory. Furthermore, the computer-readable medium may be distributed to computer systems connected over a network, and computer-readable code may be stored and executed in a distributed manner.

Examples of the program instructions include a high-level language code executable by a device for electronically processing information using an interpreter, for example, a computer, in addition to a machine code, such as that produced by a compiler.

The aforementioned hardware device may be configured to operate as one or more software modules in order to perform an operation of the present invention and vice versa.

The description of the present invention is illustrative, and a person having ordinary knowledge in the art to which the present invention pertains will understand that the present invention may be easily modified in other detailed forms without changing the technical spirit or essential characteristic of the present invention. Accordingly, it should be construed that the aforementioned embodiments are only illustrative in all aspects, and are not limitative. For example, elements described in the singular form may be carried out in a distributed form. Likewise, elements described in a distributed form may also be carried out in a combined form.

The scope of the present invention is defined by the appended claims rather than by the detailed description, and all changes or modifications derived from the meanings and scope of the claims and equivalents thereto should be interpreted as being included in the scope of the present invention.

[Industrial Applicability]

The present invention may be used for a method of searching for the endpoint of a specific data group and a data processing system therefor.

**Claims**

1. A method of searching for an endpoint of a specific group in a data set having at least one group, the method comprising steps of:

    a) receiving, by a search system, a numerical value of each of multiple individual data included in the data set;
    b) dividing, by the search system, a numerical range to which the numerical values are able to belong into a plurality of bins each having a predetermined bin width using the numerical values and generating histogram data having a number of individual data, corresponding to each of the divided bins, as a bin value;
    c) equalizing the histogram data;
    d) differentiating the equalized histogram data; and
    e) searching for a target bin satisfying a given criterion condition and present at an endpoint of a specific group based on the differentiated histogram data.

2. The method of claim 1, wherein the step of searching for a target bin satisfying a given criterion condition and present at an endpoint of a specific group based on the differentiated histogram data comprises a step of searching for, as the target bin, a bin satisfying the criterion condition based on the criterion condition in which a case where a value of a previous bin of a current bin now being searched for is smaller than a value of a subsequent bin, the value of the previous bin is equal to or smaller than 0, and the value of the subsequent bin is equal to or greater than 0 is a first bin, while searching the differentiated histogram data for a bin value of each of the bins in a given direction.

3. The method of claim 1, further comprising steps of:
    f) reducing a preset bin width by a given numerical value if the target bin satisfying the criterion condition is not searched for using the bin width; and
    performing the steps b) to e) again using the reduced bin width.

4. A method of searching for an endpoint of a specific group in a data set having at least one group, the method comprising steps of:

    a) receiving, by a search system, a numerical value of each of multiple individual data included in the data set;
    b) dividing, by the search system, a numerical range to which the numerical values are able to belong into a plurality of bins each having a predetermined bin width using the numerical values and generating histogram data having a number of individual data, corresponding to each of the divided bins, as a bin value;
    c) equalizing the histogram data;
    d) searching for a target bin satisfying a given criterion condition and present at an endpoint of a specific group based on the equalized histogram data.

5. The method of claim 4, wherein the step of searching for a target bin satisfying a given criterion condition and present at an endpoint of a specific group based on the equalized histogram data comprises a step of searching for, as the target bin, a bin satisfying the criterion condition based on the criterion condition in which a value of a previous bin of a current bin now being searched for is not a cutoff value, a value of the current bin is not a cutoff value, and a value of at least one next bin has a cutoff value, while searching the equalized histogram data for a bin value of each of the bins in a given direction.

6. A computer program which is installed in a data processing system and in which a program for executing the method according to any one of claims 1 to 5 is written.

7. A data processing system comprising:

   a processor; and
   a memory device in which software executed by the processor is stored,
   wherein the software receives a numerical value of each of multiple individual data included in a data set having at least one data group, dividing a numerical range to which the numerical values are able to belong into a plurality of bins each having a predetermined bin width using the numerical values, generates histogram data having a number of individual data, corresponding to each of the divided bins, as a bin value, and searches for a target bin present at an endpoint of a specific group based on the generated histogram data.

8. The data processing system of claim 7, wherein the software equalizes the histogram data, differentiates the equalized histogram data, and searches for the target bin, satisfying a given criterion condition and present at the endpoint of the specific group, based on the differentiated histogram data.

9. The data processing system of claim 7, wherein the software equalizes the histogram data and searches for the target bin satisfying a given criterion condition based on the equalized histogram data.

10. The data processing system of claim 7, wherein if the target bin satisfying the criterion condition is not searched for using a preset bin width, the software reduces the bin width by a given numerical value, generates a histogram again using the reduced bin width, and searches for the target bin present at the endpoint of the specific group using the re-generated histogram.

# FIG. 1

# FIG. 2

# FIG. 3

O : 1.23425, 2.13425, 4.23252, 3.13141, 1.14452 ...

# FIG. 4

Input individual data ⟞S100

Generate histogram data ⟞S110

Equalizing ⟞S120

Case ⟞S130

Search ⟞S150

Y

Differentiation ⟞S140

## INTERNATIONAL SEARCH REPORT

| International application No. |
|---|
| PCT/KR2019/005499 |

**A. CLASSIFICATION OF SUBJECT MATTER**

*G16B 40/00(2019.01)i, G16B 25/20(2019.01)i, G16B 5/20(2019.01)i*

According to International Patent Classification (IPC) or to both national classification and IPC

**B. FIELDS SEARCHED**

Minimum documentation searched (classification system followed by classification symbols)

G16B 40/00; A61B 5/055; A61B 6/03; G01N 21/958; G06K 9/00; G06T 1/00; G06T 5/40; H04N 5/208; H04N 5/21; G16B 25/20; G16B 5/20

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: data set, end point, histogram, equalization, bin

**C. DOCUMENTS CONSIDERED TO BE RELEVANT**

| Category* | Citation of document, with indication, where appropriate, of the relevant passages | Relevant to claim No. |
|---|---|---|
| A | KR 10-0442114 B1 (CHONNAM NATIONAL UNIVERSITY) 30 July 2004 See pages 3, 4; claim 1; and figure 3. | 1-10 |
| A | JP 2009-229085 A (NIKON CORP.) 08 October 2009 See the entire document. | 1-10 |
| A | US 2003-0152266 A1 (IVERS, K. T. et al.) 14 August 2003 See the entire document. | 1-10 |
| A | KR 10-2010-0101490 A (LG INNOTEK CO., LTD.) 17 September 2010 See the entire document. | 1-10 |
| A | JP 2008-534105 A (KONINKLIJKE PHILIPS ELECTRONICS N.V.) 28 August 2008 See the entire document. | 1-10 |

☐ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11 SEPTEMBER 2019 (11.09.2019) | **11 SEPTEMBER 2019 (11.09.2019)** |

| Name and mailing address of the ISA/KR | Authorized officer |
|---|---|
| Korean Intellectual Property Office Government Complex Daejeon Building 4, 189, Cheongsa-ro, Seo-gu, Daejeon, 35208, Republic of Korea | |
| Facsimile No. +82-42-481-8578 | Telephone No. |

Form PCT/ISA/210 (second sheet) (January 2015)

INTERNATIONAL SEARCH REPORT
Information on patent family members

| International application No. |
| --- |
| **PCT/KR2019/005499** |

| Patent document cited in search report | Publication date | Patent family member | Publication date |
| --- | --- | --- | --- |
| KR 10-0442114 B1 | 30/07/2004 | KR 10-2003-0043203 A | 02/06/2003 |
| JP 2009-229085 A | 08/10/2009 | None | |
| US 2003-0152266 A1 | 14/08/2003 | EP 1336852 A2 | 20/08/2003 |
| | | EP 1336852 A3 | 06/05/2004 |
| | | JP 2003-270270 A | 25/09/2003 |
| | | JP 2008-122423 A | 29/05/2008 |
| | | JP 2008-122424 A | 29/05/2008 |
| | | JP 4102214 B2 | 18/06/2008 |
| | | JP 4709239 B2 | 22/06/2011 |
| | | JP 4722953 B2 | 13/07/2011 |
| | | US 7139426 B2 | 21/11/2006 |
| KR 10-2010-0101490 A | 17/09/2010 | KR 10-1637851 B1 | 07/07/2016 |
| JP 2008-534105 A | 28/08/2008 | AT 408205 T | 15/09/2008 |
| | | CN 101151638 A | 26/03/2008 |
| | | CN 101151638 B | 16/05/2012 |
| | | EP 1869634 A2 | 26/12/2007 |
| | | EP 1869634 B1 | 10/09/2008 |
| | | JP 4991697 B2 | 01/08/2012 |
| | | US 2009-0010505 A1 | 08/01/2009 |
| | | US 8320652 B2 | 27/11/2012 |
| | | WO 2006-103594 A2 | 05/10/2006 |
| | | WO 2006-103594 A3 | 11/01/2007 |

Form PCT/ISA/210 (patent family annex) (January 2015)